# EUROPEAN PATENT SPECIFICATION

(11) **EP 1 529 848 B1**
(45) Date of publication and mention of the grant of the patent: **18.01.2006**
(21) Application number: 03090380.1
(22) Date of filing: 07.11.2003
(51) Int. Cl.: C12Q 1/68

(54) **Substances, sequences and methods for identifying epidemic methicillin resistant Staphylococcus aureus strains**
Substanzen, Sequenzen und Methoden zur Identifizierung epidemischer Methicillin-resistenter Staphylococcus aureus Stämme
Substances, séquences et méthodes pour l'identification de souches de Staphylococcus aureus épidémiques résistantes à la Méthicilline

(43) Date of publication of application: 11.05.2005
(73) Proprietor: Federal Rep. of Germany repr. by the Ministry of Health & Soc. Security, the latter repr. by the Pres. of the Robert Koch Ins., 13353 Berlin (DE)
(72) Inventor: Witte Wolfgang Prof.Dr., D-38875 Elend (DE); Strommenger Brigit, D-38855 Wemigerode (DE); Cunny Christa, D-38855 Wemigerode (DE); werner Guido Dr., D-38855 Wemigerode (DE)
(74) Representative: Bittner, Thomas L.

(56) References cited:
- US-A- 5 702 895
- FRENAY ET AL: "Molecular Typing of Methicillin-Resistant Staphylococcus aureus on the Basis of Protein A Gene Polymorphism" EUR J CLIN MICROBIOL INFECT DIS, vol. 15, no. 1, 1996, pages 60-64, XP008028927
- SABAT ET AL.: " New Method for Typing Staphylococcus aureus Strains: Multiple-Locus Variable-Number Tandem Repeat Analysis of Polymorphism and Genetic Relationships of Clinical Isolates" J CLIN MICROBIOL, vol. 41, no. 4, April 2003 (2003-04), pages 1801-1804, XP002275199
- TSUJI ET AL.: "Detection of Methicillin-Resistant Staphylococcus Aureus in Donor Eye Preservation Media by Polymerase Chain Reaction" JPN J OPHTHALMOL, vol. 42, 1998, pages 352-356, XP002275201
- HAMELS S ET AL: "CONSENSUS PCR AND MICROARRAY FOR DIAGNOSIS OF THE GENUS STAPHYLOCOCCUS, SPECIES, AND METHICILLIN RESISTANCE" BIOTECHNIQUES, EATON PUBLISHING, NATICK, US, vol. 31, no. 6, December 2001 (2001-12), pages 1364-1366,1368-1370,1372, XP001153876 ISSN: 0736-6205
- MONTESINOS ET AL: 'Epidemiologic Genotyping of Methicillin-Resistant Staphylococcus aureus by Pulsed-Field Gel Electrophoresis at a University Hospital and Comparison with Antibiotyping and Protein A and Coagulase Gene Polymorphisms' J CLIN MICROBIOL vol. 40, no. 6, June 2002, pages 2119 - 2125
- ROBERT KOCH INSTITUT: 'International einheitliche Nomenklatur epidemischer MRSA neu eingeführt' EPIDEMIOL BULLETIN vol. 27, 05 July 2002, BERLIN, D, pages 222 - 223

## Description

### Background of the Invention

Since its first identification in the early 1960s (Jevons, M. P. 1961 "Celbenin" resistant staphylococci), methicillin-resistant *Staphylococcus aureus* (MRSA) has become one of the most significant nosocmial pathogens throughout the world and it is capable of causing a wide range of hospital infections. It continues to spread through new communities wherever the methods and institutions of modem medical practice are adopted, while it regularly causes epidemics in places where it has been endemic for a decade or more. Consequently, the analysis and of the dissemination of MRSA isolates has been a research focus for a decade or more.

### Drug Resistance and Clinical Features associated with Staphylococcus aureus

*Staphylococcus aureus* has long been recognised as one of the major human pathogens responsible for a wide range of afflictions from minor infections of the skin to wound infections, bacteraemia, infections of the central nervous system, respiratory and urinary tracts, and infections associated with intravascular devices and foreign bodies. *Staphylococcus aureus* infections can be lethal. Most *Staphylococcus aureus* strains are opportunistic pathogens that can colonise individuals, without symptoms, for either short or extended periods of time, causing disease when the immune system becomes compromised. The immense genetic repertoire of this bacterium for adapting to rapidly changing and uniformly hostile environments was repeatedly shown by the emergence of *Staphylococcus aureus* strains that acquired resistance mechanisms to virtually all antimicrobial agents shortly after the introduction of these drugs into clinical practice. A study has shown that 97% of the *Staphylococcus aureus* isolates recovered carried the resistant trait to penicillin (Sa-Leao R. et al, Microb. Drug. Res. 2001; 7: 237-245).

The introduction of methicillin in clinical practice in 1960 was followed by the appearance of the first blood stream isolate of *Staphylococcus aureus* that was resistant not only to penicillin, streptomycin, and tetracycline (and occasionally to erythromycin), but to methicillin as well. Since the 1960s, MRSA strains have spread among hospitals isolates in several waves, which eventually disseminated these strains world wide.

Strain differentiation is the basis of the study of epidemiology of infectious disease. It is important for the identification of sources of (micro-) epidemics and for the identification of clones with special properties. Such as increased virulence or increased ability to spread epidemically.

The initial genetic event marking the emergence of an MRSA lineage is the acquisition of the *mec* element, introducing the methicillin-resistance determinant *mecA* into a MSSA lineage. Four types of *mec* elements or *SCCmec* have been identified so far. Type I (34 kb) was detected in the first MRSA strain isolated in 1960 in the UK (strain NCTC10442); Type II (52 kb) was detected in an MRSA strain isolated in 1982 in Japan (strain N315); Type III (66 kb) was detected in an MRSA strain isolated in 1985 in New Zealand (strain 82/2082); and type IV (20-25 kb) was identified in two community-acquired MRSA strains, as well as in representatives of the paediatric clones from Poland and from Portugal. Each of these *mec* Types gave rise to numerous MRSA clones.

In the USA an increase of MRSA in large US hospitals, from 4% in the 1980s to 50% in the late 1990s has been recorded. In some hospitals resistance frequencies as high as 80% have been recorded (Duarte C. O., The Lancet, Vol. 2, March 2003).

Particular MRSA strains are characterized by their ability to spread rapidly. These strains are called epidemic MRSA and pose a severe danger for hospitalized patients. The rapid identification of epidemic MRSA can result in a substantial benefit in combating their spread and consequently in a reduction of infection rates and finally in mortality.

Most epidemics are spread around Europe and in particular in Germany. They are designated according to multilocus sequence types by ST numbers. Representative original isolates for epidemic MRSA from Germany are Berlin epidemic strain MRSA no. 1417/97 and no. 809/96; Southern German epidemic strain MRSA no. 1155/98-2, no. 131/98; no. 2594/97; Hannover area epidemic strain MRSA no. 872/98; Northern German epidemic strain MRSA no. 134/93, no. 1450/94, no. 234/95, no. 406/98; Barnim epidemic strain MRSA no. 1678-96, no. 1293/00, No. 152-00, 633/00; Rhine-Hessen epidemic strain MRSA no. 21663, no. 2382-00 and no. 2410. These strains have arisen only recently. It is very desirable to be able to distinguish these new strains.

Strains of *Staphylococcus aureus* may be differentiated by pulsed field gel electrophoresis (PFGE). The method is labour intensive and time consuming. It involves growth of the organism, usually over night in broth, embedment of the washed cells in agarose, lysing of the cells in situ, digestion of the DNA in situ with a restriction enzyme, loading of the agar block into gel, and then separation of the fragments on the gel by PFGE. Excluding culture the whole procedure requires 2 to 5 days a period drastically to long for an urgently required *Staphylococcus aureus* identification in a hospital environment. Additionally the method is so sophisticated that an ordinary hospital will not apply it (Tang et al, J. of Clin. Microbiol. Apr. 2000, p. 1347-1351).

Strains of *Staphylococcus aureus* are traditionally differentiated by phage typing (Blair J.E., et al, Bulletin of the WHO 1961, 24: 771-784). This internationally accepted method has been helpful in elucidating hospital epidemics of *Staphylococcus aureus,* now and in the past (Vandenbrouck-Grauls C. M. et al, Eur. J. of Clin Micr. & Inf. Dis. 1991, 10: 6-11). Some *Staphylococcus aureus* strains are susceptible to only one or two phages of the international standard set, or may be completely unsusceptible to those phages (Zierdt C. H. et al J. of Clin. Micr. 1992, 30: 252-254). Especially, *Staphylococcus aureus* strains sensitive to only one or two phages may erroneously appear to be identical on the basis of phage typing.

Rapid and reliable strain differentiation is important for the identification of sources of (micro-) epidemics in particular in hospitals. It is very important to be able to identify clones with special properties, such as increased virulence or increased ability to spread epidemically.

Previous attempts of identifying a nucleic acid sequence that is useful for unambiguous and rapid strain differentiation have failed. Some newer, also commercial methods use DNA sequencing. However, this is tedious and time consuming. In Frénay et al (Eur. J. Clin. Microbiol. Infect. Dis., 1996, 15:60-64) it was observed, that when analyzing the spa-type (i.e. the X-region), strains isolated in a single hospital or even in a single patient differed. So far spa-typing has not been an alternative, due the fact that unambiguous identification was not possible. Even though spa typing is known in the state of the art as a method for identifying *Staphylococcus aureus,* it has never been used on its own for the identification of specific strains. A former document in fact disclosed that sequences derived from this region were not strain specific. The authors do state that the X-region is stable *in vitro* and *in vivo* (Frénay et al, Eur. J. Clin. Microbiol. Infect. Dis., 1996, 15:60-64). So far spa-typing has not been an alternative, due the fact that unambiguous identification was not possible.

Consequently, it would be desirable to have sequences, substances and methods for identifying such novel *Staphylococcus aureus* strains in a manner more discriminatory and faster than known in the art. Ideally such sequences, substances and methods would make it possible to perform the analysis in a reliable manner within a standard hospital environment. Furthermore, such sequences, substances and methods would not need the culturing for a prolonged period of time of the *Staphylococcus aureus* isolates.

### Detailed Description of the Invention

Unexpectedly the inventors are able to use the nucleic acid molecules according to the invention which have now been discovered in order to unambiguously identify various *Staphylococcus aureus* strains, thus making phage typing and/or pulsed field gel electrophoresis obsolete.

The present invention relates to a novel nucleic acid molecule which is selected from the group of SEQ ID NOs: 1, 2, 3, 4, 5, 6, 7, 8, 9, 10, 11, 12, 13, 14, 15, 16, 17, 18, 19 and 20 or a reverse complement thereof (Table 1 depicts the numbers and names of the corresponding sequences as well as the strain number for which the sequence is specific). In the context of this invention the nucleic acids according to the invention serve to unambiguously identify the corresponding strain.

The term nucleic acid molecule and oligonucleotide herein refers to primers, probes, samples, and oligonucleotide fragments as well as other forms of nucleic acid molecules. The term nucleic acid molecule and oligonucleotide is further generic for polydeoxribonucleotides (containing 2-deoxy-D-ribose) and for polyribonucleotides (containing D-ribose) or to any other form of polynucleotide, which is an N-glycosid of a purin base or a pyrimidin base or a modified purin base or pyrimidin base. The terms equally encompass polyamid with a purin or pyrimidin base also referred to as PNA's. Herein nucleic acid molecule and oligonucleotide shall both refer to molecules of any given length and shall be interpreted to mean a certain length. The nucleic acid molecule and oligonucleotide referred to herein may be present in singly, double or triple stranded form or as a PCR product, cDNA, RNA, mRNA or PNA.

The inventors have found that unlike most other spa-sequences disclosed herein stemming from *Staphylococcus aureus* it is possible to use the sequences according to the invention to unambiguously identify certain *Staphylococcus aureus* strains (Table 1).

**Table 1:**

| | |
|---|---|
| SEQ ID NO. 1 | Berlin epidemic strain no. 1417/97 (Int. no. ST 45) |
| SEQ ID NO. 2 | Berlin epidemic strain no. 809/96 (Int. no. ST 45) |
| SEQ ID NO. 3 | Southern German strain no. 1155/98-2 (Int. no. ST 228) |
| SEQ ID NO. 4 | Southern German strain no. 131/98 (Int. no. ST 228) |
| SEQ ID NO. 5 | Southern German strain no. 2594/97 (Int. no. ST 288) |
| SEQ ID NO. 6 | Hannover area strain no. 872/98 (Int. no. ST 254) |
| SEQ ID NO. 7 | Northern German strain no. 134/93 (Int. no. ST 247) |
| SEQ ID NO. 8 | Northern German strain no. 1450/94 (Int. no. ST 247) |
| SEQ ID NO. 9 | Northern German strain no. 234/95 (Int. no. ST 247) |
| SEQ ID NO. 10 | Northern German strain no. 406/98 (Int. no. ST 247) |
| SEQ ID NO. 11 | Barnim strain no. 1678/96 (Int. no. ST 22) |
| SEQ ID NO. 12 | Barnim strain no. 1293/00 (Int. no. ST 22) |
| SEQ ID NO. 13 | Barnim strain no. 152/00 (Int. no. ST 22) |
| SEQ ID NO. 14 | Barnim strain no. 633/00 (Int. no. ST 22) |
| SEQ ID NO. 15 | Rhine Hesse no. 2163/00 (Int. no. ST 5) |
| SEQ ID NO. 16 | Rhine Hesse no. 2382/00 (Int. no. ST 5) |
| SEQ ID NO. 17 | Rhine Hesse no. 2410/00 (Int. no. ST 5) |
| SEQ ID NO. 18 | Hannover no. 733/96 (Int. no. ST 254) |
| SEQ ID NO. 19 | Hannover no. 1000/93 (Int. no. ST 254) |
| SEQ ID NO. 20 | Hannover no. 1442/93 (Int. no. ST 254) |

A preferred embodiment of the invention relates to certain parts of the complete sequences according to the SEQ ID NOs: 1, 2, 3, 4, 5, 6, 7, 8, 9, 10, 11, 12, 13, 14, 15, 16, 17, 18, 19 and 20 or the reverse complements thereof (Table 2).

In a further embodiment of the invention the nucleic acid molecules with the nucleotide sequences set forth in SEQ ID NOs: 1, 2, 3, 4, 5, 6, 7, 8, 9, 10, 11, 12, 13, 14, 15, 16, 17, 18, 19 and 20 or the reverse complements thereof may be used to identify the following *Staphylococcus aureus* strains:

So far no system is available that makes it possible to identify the following strains *Staphylococcus aureus* rapidly and unambiguously.
a) the Berlin epidemic strain MRSA no. 1417/97 and no. 809/96 (international no. ST 45);
b) the Southern German epidemic strain MRSA no. 1155/98-2, no. 131/98; no. 2594/97 (international no. 228);
c) the Hannover area epidemic strain MRSA no. 872/98 (international no. ST 254);
d) the Northern German epidemic strain MRSA no. 134/93, no. 1450/94, no. 234/95, no. 406/98 (international no. ST 247);
e) the Barnim epidemic strain MRSA no. 1678-96, no. 1293/00, No. 152-00, 633/00 (international no. ST 22) and;
f) the Rhine Hesse epidemic strain MRSA no. 21663, no. 2382-00 and no. 2410 (international No. ST 5).

In one embodiment of the invention it relates to an oligonucleotide or a molecule comprising an oligonucleotide, for the detection of one of the nucleic acid molecules according to the invention as set out above. This oligonucleotide is selected from the group comprising the nucleotide sequences according to SEQ ID NOs: 21, 22, 23, 24 , 25, 26, 27, 28 and 29 (see also Table 2), or the reverse complementary sequences thereof. One skilled in the art will appreciate that the oligonucleotides may also differ in sequence slightly for those mentioned herein. Thus, one can envision oligonucleotides that are slightly longer or slightly shorter than those disclosed according to SEQ ID NOs: 21, 22, 23,24 ,25, 26, 27, 28 and 29. Also one or a few nucleotides may differ. It is however important that the specificity of the oligonucleotides is maintained and the melting temperature not altered significantly. That means that if the oligonucleotide is altered in sequence the melting temperature is preferentially maintained. The melting temperature is calculated as follows G/C (=4°C) and A/T (=2°C). One skilled in the art will equally recognize that also modified nucleotides may form part of or all of an oligonucleotide according to the invention.

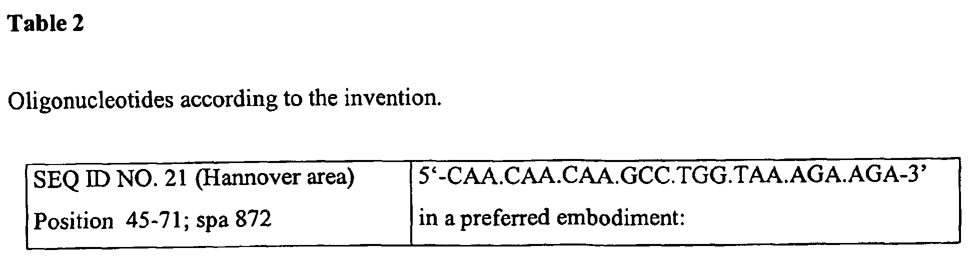

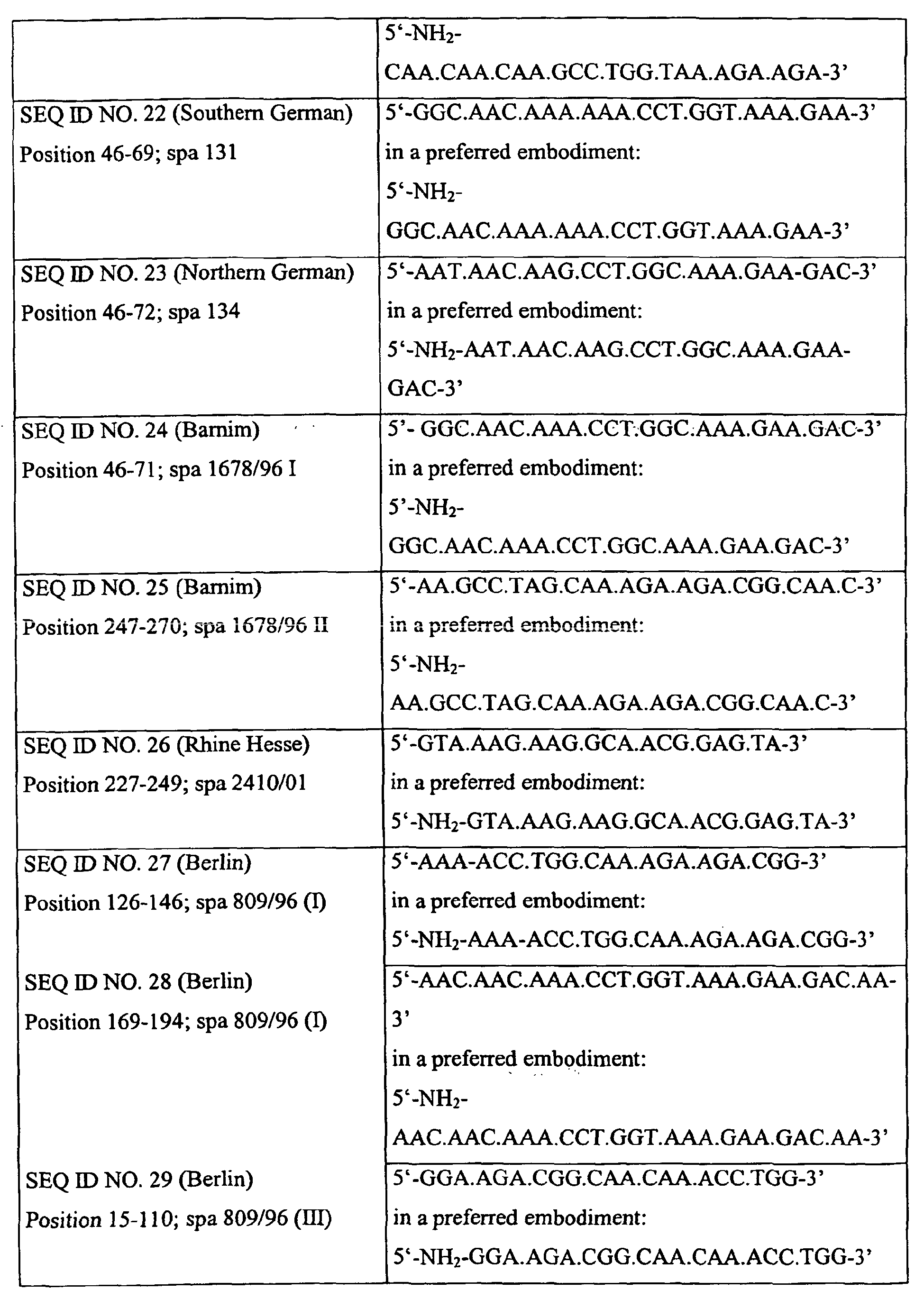

As can be seen above, in a preferred embodiment the oligonucleotides are modified at the 5'-end with an amino group for binding to surfaces as e.g. capture probes. In one embodiment the oligonucleotides as listed in table 2 are used with a 40-mer poly T tail linked to the 5'-end and spotted either on glass slides, nylon or another solid support. This tail may also be between 2 and 50 nucleotides and consist of poly A, C, G, or T or a mixture thereof. Longer tails are possible however not ideal.

In one embodiment the oligonucleotide comprising a chemical modification is selected from the group comprising a modification which serves to immobilize the oligonucleotide and a modification which serves to label the oligonucleotide.

In a preferred embodiment of the invention the oligonucleotide additionally may comprise a chemical modification or a label selected from the group comprising an amino linker, a fluorescent dye such as fluorescein or a cyanin dye, biotin, digoxigenin or other modifications well known to those skilled in the art. The modification may serve to enable binding to a solid phase or serve to incorporate a label. For example a biotin labelled oligonucleotide can be detected with an enzyme that is coupled to streptavidin. Such an enzyme may be alkaline phosphatase or peroxidase. The reaction will subsequently result in a colour change. The modification may also be a linker such as an amino-linker. The label may equally be a radioisotope such as ¹²⁵I, ³⁵C, ³²P or ³⁵P. Usually the modification or label is covalently bound to the oligonucleotide. Enzymes which may used in combination with a modification in order to detect the oligonucleotide according to the invention are e.g. peroxidase, hydrolase, lyase, oxido-reductase, transferase, isomerase and ligase. Such methods are known to the skilled artisan ((Wetmur JG. Crit Rev Biochem Mol Biol 1991; (3-4): 227-59; Temsamani J. et al. Mol Biotechnol 1996 Jun;5(3):223-32). In some cases where enzymes react as conjugates colour changing ubstances must be present (Tijssen, P. *Practice and Theory of Enzyme Immunoassays in Laboratory Techniques in Biochemistry and Molecular Biology,* eds. R. H. Burton and P.H. van Knippenberg (1998). Labels or modifications may be added to the oligonucleotide by using e.g. a T4 polynucleotide kinase or a terminal transferase. These enzymes may incorporate e.g. a fluorescent dye or a radioactive dye. For this invention it is necessary that either the nucleic acids according to the invention are detectable or the oligonucleotides according to the invention. Labelling may be done according to the prior art such as disclosed in US patent number 6,037,127.

In a particularly preferred embodiment of the invention the oligonucleotide is immobilized on a solid phase. The inventors have shown that particularly good results are achievable when the oligonucleotide according to the invention is immobilized and the nucleic acid molecule according to the invention is in solution when the hybridization reaction is performed. The oligonucleotide is preferentially immobilized on one of the following solid phases, nylon, nitrocellulose, polystyrol, silicates such as glass. The solid phase may also be a strip or a microtiter plate. Ordinarily the oligonucleotide is bound by either its 5'-end or its 3'-end whereby the molecule is free to sway in solution. Those skilled in the art know various means of attaching an oligonucleotide to a solid phase. This can be done by means of a modification such as an amino-linker, a thiol group, carboimide, succinimide or another modification. Preferred is a covalent bond however, non covalent binding is equally possible. In the later case materials such as charged or uncharged nylon, nitrocellulose, cellulose acetate, are used. The solid phase may be coated with a substance which can facilitate binding of the oligonucleotide such as avidin which will bind a biotin modified oligonucleotide. In a preferred embodiment the solid phase is micro array consisting of a silicate material such as glass. Numerous types of micro arrays are known to the artisan (McGlenn et al, (2001) Miniaturization technologies for molecular diagnostics. Clin. Chem. 47(3), 393-402). The oligonucleotide may be attached to the solid phase with an additional intervening linker. Such a linker is for example a polynucleotide either a homopolymer, such as multiple Adenin nucleotides or a heteropolymer wherein a nucleotide sequence of varying composition is chosen that will not interfere with hybridization.

The invention further relates to a method of identifying a clinically relevant *Staphylococcus aureus* strain comprising at least the step of detecting a nucleic acid molecule according to the invention by means of hybridization with a sequence specific probe such as the oligonucleotides according to the invention under stringent conditions.

The clinically relevant *Staphylococcus aureus* strains according to the invention comprise but are not limited to:
a) the Berlin epidemic strain MRSA no. 1417/97 and no. 809/96 (international no. ST 45);
b) the Southern German epidemic strain MRSA no. 1155/98-2, no. 131/98; no. 2594/97 (international no. 228);
c) the Hannover area epidemic strain MRSA no. 872/98 (international no. ST 254);
d) the Northern German epidemic strain MRSA no. 134/93, no. 1450/94, no. 234/95, no. 406/98 (international no. ST 247);
e) the Barnim epidemic strain MRSA no. 1678-96, no. 1293/00, No. 152-00, 633/00 (international no. ST 22) and;
f) the Rhine Hesse epidemic strain MRSA no. 21663, no. 2382-00 and no. 2410 (international No. ST 5).

Hybridization probes act by forming selectively duplex molecules with complementary stretches of a sequence of a gene or a sample or a cDNA. The selectivity of the process can be controlled by varying the conditions of hybridization.

Stringent conditions for the hybridization will be used, e.g. low salt in the range of 0.02 M to 0.15 M salt and/or high temperatures in the range from 50°C degrees centigrade to 70°C degrees centigrade. Stringency can be further improved by the addition of formamide to the hybridization solution. The use of stringent conditions which means that only little mismatch or a complete match will lead to a hybridization product is important for the invention. Thus, as used herein stringent hybridization conditions are those where between 0.02 M to 0.15 M salt and/or high temperatures in the range from 50°C degrees centigrade to 70°C degrees centigrade are applied and where long probes are used, *i.e.* probes that are longer than 30 nucleotides.

The use of highly stringent conditions or conditions of "high stringency" means that only very little mismatch or a complete match will lead to a hybridization product. Thus, as used herein highly stringent hybridization conditions are those where between 0.02 - 0.3 M salt and 65°C degrees centigrade are applied for about 5 to 18 hours of hybridization time and additionally, the sample filters are washed twice for about 15 minutes each at between 60°C - 65°C degrees centigrade, wherein the first washing fluid contains about 0.1 M salt (NaCl and/or Sodium Citrate) and the second contains only about 0.02 M salt (NaCl and/or Sodium Citrate).

In a preferred embodiment the following conditions are considered to be highly stringent:

Hybridisation in a buffer containing 2 x SSC (0.03 M Sodium Citrate, 0.3 M NACl) at 65°C - 68°C degrees centigrade for 12 hours, followed by a washing step for 15 minutes in 0.5 x SSC, 0.1% SDS, and a washing step for 15 minutes at 65°C degrees centigrade in 0.1 x SSC, 0.1% SDS.

In a very preferred embodiment hybridization is performed with the oligonucleotides according to the invention in an H1-buffer (5x puffer H1: 0.55 g Na₂HPO₄, 0.1 g NaH₂PO₄, 0.625 ml 20 x SSC, 0.045 g EDTA, 0.8 g SPS, 0.25 ml Aq. bidest) at a temperature between 35°C and 45°C most preferentially at 42°C for 1 to 8 hours most preferentially for 4 hours. Washing is performed in 1 X SSC to 3 X SSC, most preferentially in 2 X SSC with 0.1% SDS to about 3% SDS, most preferentially 0.5% SDS at about room temperature

Less stringent hybridization conditions, e.g. 0.15 M salt - 1 M salt and/or temperatures from 22°C degrees centigrade to 56°C degrees centigrade do not lead to good results.

Unspecific hybridization products are removed by washing the reaction products repeatedly in 2 x SSC solution and increasing the temperature.

In a preferred embodiment of the invention the sequence specific probe is one or more of the oligonucleotides according to the invention (SEQ ID Nos. 21-29).

In one embodiment DNA extraction and amplification is performed prior to hybridization. This can be done be means known to those skilled in the art such as with the DNeasy Tissue Kit (Qiagen, Hilden, Germany) following the manufacturer's instructions and using lysostaphin (100 µg/ml, Sigma, Germany) to achieve bacterial lysis.

In one embodiment more than one oligonucleotide according to the invention are used in one reaction simultaneously. The great strength of the invention lies in the fact that it has been possible to use all oligonucleotides in one reaction under conditions which work equally well for all oligonucleotides according to the invention.

In one embodiment the invention is performed on a dry rapid test. Here the invention contains a chromatographic material that comprises a sample reception zone, a separation zone including a binding area, in which one or more sequence-specific nucleic acids are immobilized, and a zone which absorbs a liquid downstream of the separation zone including a binding area. In this embodiment, the sequence-specific nucleic acid probes are immobilized via a polymer linker. The method comprises the following steps i) denaturing, in the case of double-stranded nucleic acids, the nucleic acid to be detected and then neutralizing it, ii) applying the nucleic acid to be detected to the sample reception zone in a run buffer which contains mildly denaturing agents, iii) the nucleic acid moving from the sample reception zone in direction of the liquid absorbing zone, (iv) contacting the nucleic acid to be detected in the binding area of the separation path with the sequence-specific nucleic acid probe and hybridizing it with the sequence-specific nucleic acid probe, v) detecting the nucleic acid or the hybridization of the nucleic acid with the sequence specific nucleic acid probe via a label that is attached to the nucleic acid to be detected or via the detection of a label of the nucleic acid double strand (WO 03/039703).

In a preferred embodiment of the invention the method according to the invention is used in order to detect a nucleic acid and/or an organism chosen from the group comprising the Berlin epidemic strain MRSA no. 1417/97 and no. 809/96 (international no. ST 45), the Southern German epidemic strain MRSA no. 1155/98-2, no. 131/98; no. 2594/97 (international no. 228), the Hannover area epidemic strain MRSA no. 872/98 (international no. ST 254), the Northern German epidemic strain MRSA no. 134/93, no. 1450/94, no. 234/95, no. 406/98 (international no. ST 247), the Barnim epidemic strain MRSA no. 1678-96, no. 1293/00, No. 152-00, 633/00 (international no. ST 22) and, the Rhine Hesse epidemic strain MRSA no. 21663, no. 2382-00 and no. 2410 (international No. ST 5).

Ordinarily the method according to the invention will comprise a PCR amplification reaction prior to hybridization, wherein the nucleic acid sequence according to the invention or a portion thereof is amplified and sequence specific primers are applied. PCR reactions are known to those skilled in the art. Some details are given below.

In such a PCR amplification reaction the following steps are performed a) combining the sample containing the target region, here the nucleic acid molecule according to the invention, with at least on nucleotide triphosphate, a thermally stable polymerase, a buffer and two sequence specific primers, b) exposing the reaction mixture to at least one temperature cycle including at least a high temperature denaturation phase and a lower temperature extension phase, and thereby producing at least a partially amplified product. Ideally the cycles are repeated and thus a larger quantity of the product of desire is created.

In this method according to the invention it is preferred that the primers according to the invention comprises preferably between 3 and 60 nucleotides more preferably between 10 and 50 nucleotide most preferably between 12 and 25 nucleotides for each nucleic acid moiety. It is essential in this context that the primers are of sufficient length to bind the target nucleic acid molecule with sufficient stringency. PNA-DNA hybrid oligonucleotides (see Finn, P. J. et al., N.A.R. 24, 3357-3363 (1996), Koch, T. et al., Tetrahedron Letters 36, 6933-6936 (1995), Stetsenko, D. A. et al., Tetrahedron letters 37, 3571-3574 (1996), Bergmann, F et al., Tetrahedron Letters 36 6823-6826 (1995) and Will, D. W. et al., Tetrahedron 51, 12069-12082 (1995)) are also considered as primers for the process of the present invention.

As a thermally stable polymerase, a DNA polymerase may be selected from the group comprising *Taq* DNA polymerase, *Tth* DNA polymerase or Klentaq (Taq DNA polymerase (-exo5'-3'), Korolev et al., (1995) Proc. Natl. Acad. Sci. USA 92, 9246-9268. The use of Taq DNA polymerase in the method of the present invention is especially preferred. One skilled in the art will recognize that numerous different thermally stable polymerases may be used.

Alternatively as a thermally stable polymerase, a DNA polymerase which has a decreased discrimination against labelled nucleotides may be used.

The number of thermal cycles may range from about 10 to about 45 depending on the amount of template DNA and its purity. As the availability of the nucleic acid according to the invention is high in the method according to the invention the cycle period may be short.

Routinely, cycling consists of (i) a denaturing cycle, (ii) an annealing cycle and (iii) an extension cycle. Alternatively, only two cycles may be applied, (i) a denaturing cycle and (ii) an annealing and extension cycle.

Preferably the denaturing cycle is performed at between 100°C and 85°C, more preferably at between 98°C and 90°C, most preferably at between 96°C and 92°C.

Preferably the annealing cycle is performed at between 80°C and 45°C, more preferably at between 70°C and 50°C, most preferably at between 60°C and 55°C.

Preferably the extension cycle is performed at between 80°C and 50°C, more preferably at between 75°C and 60°C, most preferably at between 73°C and 68°C.

Preferably the denaturing cycle is performed for 3 minutes, more preferably for 30 seconds.

Preferably the annealing cycle is performed for 3 minutes, more preferably for 30 seconds. Preferably the extension cycle is performed for 4 minutes, more preferably for 30 seconds, however the extension time vary depending on the length of the template, in particular the extension time may be raised if the template length increases.

Buffers components which can be used can include, but are not limited to, Tris-HCl at a pH of about 7.0 to 10 and concentration of about 2 to 60 mM, ammonium sulfate at a concentration of about 10-20 mM, preferably 15 mM, MgCl₂ at a concentration of about 1 to 10 mM, and optionally, about 0.05 mM mercaptoethanol, about 0.28% Tween® 20 and/or about 0.02% Nonidet® 40.

Nucleotide triphosphates are preferably deoxynucleotides and can be selected from, but are not limited to, dGTP, dATP, dTTP and dCTP. In addition, derivatives of deoxynucleotides, which are defined as those deoxynucleotides which are capable of being incorperated by a thermally stable DNA polymerase into nascent DNA molecules synthesized in the thermal cycling reaction, can also be used according to the invention. Such derivatives include, but are not limited to thionucleotides, 7-deaza-2'-dGTP, 7-deaza-2'-dATP as well as deoxyinosine triphosphate which may also be used as a replacement deoxynucleotide for dATP, dGTP, dTTP or dCTP. The above mentioned deoxynucleotides and derivatives thereof are preferably used at a concentration of about 50 µM to about 4 mM.

In a preferred embodiment one or more of the nucleotides incorporated are labelled. For example, single and differential labels may consist of the group comprising enzymes such as β-galactosidase, alkaline phosphatase and peroxidase, enzyme substrates, coenzymes, dyes, chromophores, fluorescent, chemiluminescent and bioluminescent labels such as FITC, Cy5, Cy5.5, Cy7, Texas-Red and IRD40(Chen et al. (1993), J. Chromatog. A 652: 355-360 and Kambara et al. (1992), Electrophoresis 13: 542-546), ligands or haptens such as biotin, and radioactive isotopes such as ³H, ³⁵S, ³²P ¹²⁵I and ¹⁴C.

In one embodiment of the method of the invention, the nucleic acid molecule to be amplified can be present in the form of total genomic DNA. Preferably, the genomic DNA has a length greater than or equal to 2 kb. Generally all forms of template may be used, *e.g*. purified nucleic acids, *i.e.* nucleic acids where one fraction may be enriched or not, one example being plasmid DNA the other purified genomic DNA. The method may also be performed and this preferred by simply adding *Staphylococcus aureus* cells or rather the sample taken directly from the patient to the PCR reaction. It is also possible to grow the sample taken from the patient on [x] plates before an aliquot is added from this plate to the directly to the PCR reaction mix.

In a preferred embodiment the PCR reactions are performed with Ready-to-go PCR beads (Amersham Pharmacia Biotech, Freiburg, Germany) in a 25 µl reaction containing approximately 10 ng of template DNA and 2.5 pmol of each primer. Initial denaturation at 94°C for 3 min is followed by 30 cycles of amplification with 94°C for 30 sec, annealing at 55°C for 30 sec and extension at 72°C for 30 sec (except for the final cycle with an extension step of 4 min).

Here, PCR products are labelled by either using digoxigenin-labelling for hybridization to nitrocellulose bound capture probes or by random primed labelling with fluorescein and use of the Flourescein High Prime* kit (Roche, Mannheim, Germany) in case of microarrays.

In a very preferred embodiment the method according to the invention is performed with primers consisting essentially of the following nucleotide sequence a) PRIMER 1 (SEQ ID NO. 30): 5'-CAA GCA CCA AAA GAG GAA-3', b) PRIMER 2 (SEQ ID NO. 31): 5'-CAC CAG GTT TAA CGA CAT '-3' and optionally the PCR product is labelled during the amplification reaction by incorporation of a labelled nucleotide, or the PCR product is labelled after the PCR reaction prior to hybridization and optionally one or both of the Primers are labelled prior to the initiation of the PCR reaction.

In case of binding to nitrocellulose and labelling of the PCR product the Nucleic Acid Detection kit from Roche (Mannheim, Germany) is used in a preferred embodiment. In case of spotting onto glass slides and labelling of the PCR product with fluorescein, detection can be performed on a array Wox biochip reader (Applied Precision, Marlborough, UK) and use of the Alexa 488 nm filter.

The invention also covers a device for identifying a clinically relevant *Staphylococcus aureus* strain comprising a solid phase with one or more oligonucleotides according to the invention. The device may additionally comprise a solid phase and or an apparatus for detecting the nucleic acid molecules according to the invention. In a preferred embodiment the device comprises a solid phase to which the nucleic acids according to the invention are fixed. Such a device may comprise a glass plate, a nylon membrane another membrane capable of binding nucleic acids, a rapid dry test membrane as disclosed in WO 03/039703 The solid phase may chosen from the group comprising a nucleic acid array, membrane, nitrocellulose, charged nitrocellulose, uncharged nitrocellulose, nylon membrane, charged nylon membrane, uncharged nylon membrane, glass array.

The invention equally covers a kit for identifying a clinically relevant *Staphylococcus aureus* strain at least comprising an oligonucleotide according to the invention or a device according to the invention. The kit may also comprise other reagents or enzymes such as buffers, nucleotides or the like.

While the foregoing has been set forth in detail, the Examples are presented for elucidation, and not limitation. Modifications and improvements on the compound and or process according to the invention disclosed above which are within the purview and abilities of those in the art are included within the scope of the claims.

In the examples:
Figure 1

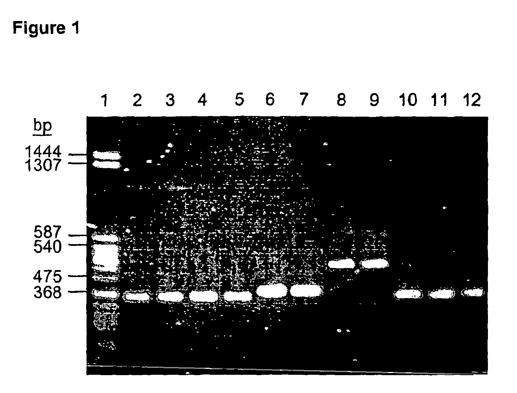

   Figure 1 shows amplimers of the X-Region of *spa* for different MRSA epidemic strains wherein, lanes 2 and 3 show the Berlin epidemic strain (1155/93, 809/96), lanes 4 and 5 show the southern German epidemic strain (538/95, 131/98), lanes 6 and 7 show the northern German epidemic strain (134/93, 1450/94), lanes 8 and 9 show the Barnim epidemic strain (1678/96, 2378/00) and lanes 10, 11 and 12 show the Hannover area epidemic strain (1000/93, 872/00).
Figure 2

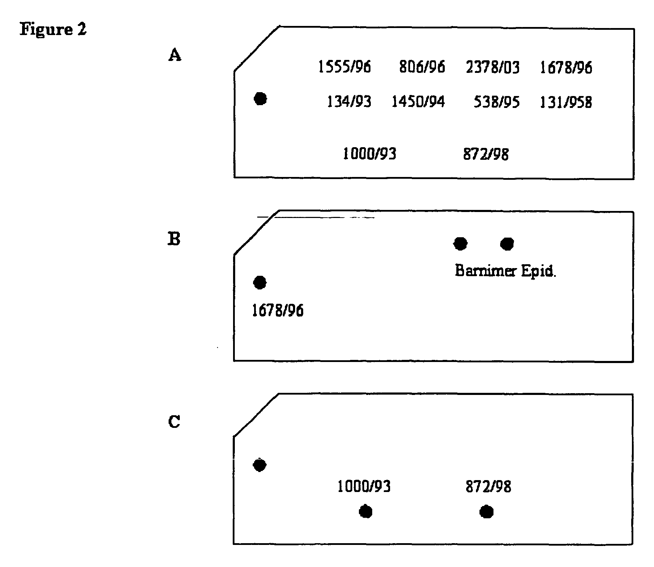

   Figure 2 shows Digoxigenin labbeling and exposure with alkaline phosphatase was perfomed as outlined in experiment 1. The figure shows that the positive control gives the expected positive result and the strains Barnim MRSA as well as Hannover area epidemic strain are detected with great specificity. Figure 2 A shows the location of probes according to the invention on the nylon membrane below. The relevant Staphylococcus aureus strain that is to be detected is shown as a strain number. Figure 2 B shows results of the application of the invention to the detection of the Barnim MRSA strain. Figure 2 C shows results of the application of the invention to the detection of the Hannover area epidemic strain.

### Example 1

Initially DNA extraction was performed as follows: DNA was extracted from about 10 single colonies grown on blood agar at 37°C for about 18 hours with the DNeasy Tissue Kit (Qiagen, Hilden, Germany) following the manufacturer's instructions and using lysostaphin (100 µg/ml, Sigma, Germany) to achieve bacterial lysis. The primers 1 and 2 were used (SEQ ID NO. 30: 5'-CAA GCA CCA AAA GAG GAA and SEQ ID NO: 31: 5'-CAC CAG GTT TAA CGA CAT). PCR reactions were performed with Ready-to-go PCR beads (Amersham Pharmacia Biotech, Freiburg, Germany) in a 25 µl reaction containing approximately 10 ng of template DNA and 2.5 pmol of each primer. Initial denaturation at 94°C for 3 min was followed by 30 cycles of amplification with 94°C for 30 sec, annealing at 55°C for 30 sec and extension at 72°C for 30 sec (except for the final cycle with an extension step of 4 min). PCR products were labelled by either using digoxigenin-labelling for hybridization to nitrocellulose bound capture probes or by random primed labelling with fluorescein and use of the Flourescein High Prime* kit (Roche, Mannheim, Germany) in case of microarrays.

Capture probes as listed in table 2 were used with a 40 mer poly T tail linked to the 5'-end and spotted either on glass slides or onto nylon. Hybridization and washing was done in an H1-buffer at 42°C for 4 hours, washing in 2 x SSC with 0.5% SDS at room temperature.

Detection was done with the Nucleic Acid Detection kit from Roche (Mannheim, Germany). In case of spotting onto glass slides and labelling detection was performed on a array Wox biochip reader (Applied Precision, Marlborough, UK) and use of the Alexa 488 nm filter.

The features disclosed in this specification, claims and/or the figures may be material for the realization of the invention in its various embodiments, taken in isolation or in various combinations thereof.

## Claims

1. A nucleic acid molecule which is selected from the group of SEQ ID NOs: 1, 2, 3, 4, 5, 6, 7, 8, 9, 10, 11, 12, 13, 14, 15, 16, 17, 18, 19 and 20 or a reverse complement thereof.

2. The nucleic acid molecule of claim 1 which is genomic DNA, cDNA, a PCR product or RNA.

3. An oligonucleotide or a molecule comprising an oligonucleotide, for the detection of one of the nucleic acid molecules according to claims 1 or 2, selected from the group of nucleotide sequences as set forth in SEQ ID NOs: 21, 22, 23, 24, 25, 26, 27, 28, and 29 or the reverse complementary sequence thereof.

4. An oligonucleotide according to claim 3, additionally, comprising a chemical modification selected from the group comprising a modification which serves to immobilize the oligonucleotide and a modification which serves to label the oligonucleotide.

5. An oligonucleotide according to claims 3 or 4 wherein, the oligonucleotide is immobilized on a solid phase.

6. Method of identifying a clinically relevant *Staphylococcus aureus* strain comprising at least the step of detecting a nucleic acid molecule according to claim 1 by means of hybridization with a sequence specific probe under stringent conditions.

7. Method of claim 6 wherein, the sequence specific probe is an oligonucleotide according to claims 3 to 5.

8. Method according to claim 6 or 7 wherein, the hybridization is performed with a chromatographic material that comprises (i) a sample reception zone, (ii) a separation zone including a binding area, in which one or more sequence-specific nucleic acids are immobilized, (ii) and a zone which absorbs a liquid downstream of the separation zone including a binding area, (iii) and the sequence-specific nucleic acid probes are immobilized via a polymer linker and wherein the method comprises the following steps:
a) denaturing, in the case of double-stranded nucleic acids, the nucleic acid to be detected and then neutralizing it,
b) applying the nucleic acid to be detected to the sample reception zone in a run buffer which contains mildly denaturing agents,
c) the nucleic acid moving from the sample reception zone in direction of the liquid absorbing zone,
d) contacting the nucleic acid to be detected in the binding area of the separation path with the sequence-specific nucleic acid probe and hybridizing it with the sequence-specific nucleic acid probe,
e) detecting the nucleic acid or the hybridization of the nucleic acid with the sequence specific nucleic acid probe via a label that is attached to the nucleic acid to be detected or via the detection of a label of the nucleic acid double strand.

9. Method of claim 6 to 8 wherein, the *Staphylococcus aureus* strain that is to be identified is chosen from the group comprising the epidemic strains:
a) the Berlin epidemic strain MRSA no. 1417/97 and no. 809/96 (international no. ST 45);
b) the Southern German epidemic strain MRSA no. 1155/98-2, no. 131/98; no. 2594/97 (international no. ST 228);
c) the Hannover area epidemic strain MRSA no. 872/98 (international no. ST 254);
d) the Northern German epidemic strain MRSA no. 134/93, no. 1450/94, no. 234/95, no. 406/98 (international no. ST 247);
e) the Barnim epidemic strain MRSA no. 1678-96, no. 1293/00, No. 152-00, 633/00 (international no. ST 22) and;
f) the Rhine Hesse epidemic strain MRSA no. 21663, no. 2382-00 and no. 2410 (international No. ST 5).

10. Method according to claims 6 to 9 wherein, prior to hybridization a PCR amplification reaction is performed, wherein the sequence according to claim 1 or a portion thereof is amplified and sequence specific primers are applied.

11. Method according to claim 10 wherein, the PCR amplification is performed with sequence specific primers consisting essentially of the following nucleotide sequence: and optionally the PCR product is labelled during the amplification reaction by incorporation of a labelled nucleotide, or the PCR product is labelled after the PCR reaction prior to hybridization and optionally one or both of the Primers are labelled prior to the initiation of the PCR reaction.

12. Device for identifying a clinically relevant *Staphylococcus aureus* strain comprising a solid phase with one or more immobilized nucleic acid molecules according to claims 3 or 4.

13. Device according to claim 12 wherein, wherein the solid phase is selected from the group comprising nucleic acid array, membrane, nitrocellulose, charged nitrocellulose, uncharged nitrocellulose, nylon membrane, charged nylon membrane, uncharged nylon membrane, glass array.

14. Kit for identifying a clinically relevant *Staphylococcus aureus* strain comprising a nucleic acid molecule according to claims 3 to 5 or a device according to claims 12 to 13.

## Patentansprüche

1. Nukleinsäuremolekül ausgewählt aus der Gruppe von, SEQ ID NOs: 1, 2, 3, 4, 5, 6, 7, 8, 9, 10, 11, 12, 13, 14, 15, 16, 17, 18, 19 und 20, oder ein reverses Komplement davon.

2. Nukleinsäuremolekül nach Anspruch 1, welches DNA, cDNA, ein PCR Produkt oder RNA ist.

3. Oligonukleotid oder ein Molekül umfassend ein Oligonukleotid, zur Detektion eines der Nukleinsäuremoleküle nach Anspruch 1 oder 2, ausgewählt aus der Gruppe wie dargelegt in SEQ ID NOs: 21, 22, 23, 24, 25, 26, 27, 28, und 29, oder die reverskomplementäre Sequenz davon.

4. Oligonukleotid nach Anspruch 3, zusätzlich umfassend, eine chemische Modifikation ausgewählt aus der Gruppe umfassend, Modifikationen die zur Immobilisierung des Oligonukleotides dienen und Modifikationen die zur Markierung des Oligonukleotids dienen.

5. Oligonukleotid nach Anspruch 3 oder 4, wobei das Oligonukleotid auf einer festen Phase immobilisiert ist.

6. Verfahren zur Identifikation eines klinisch relevanten *Staphylococcus aureus* Stammes umfassend wenigstens den Schritt der Detektion eines Nukleinsäuremoleküls nach Anspruch 1 mittels Hybridisierung mit einer sequenzspezifischen Probe unter stringenten Bedingungen.

7. Verfahren nach Anspruch 6 wobei die sequenzspezifische Probe ein Oligonukleotid nach Ansprüchen 3 bis 5 ist.

8. Verfahren nach Anspruch 6 oder 7 wobei die Hybridisierung mit einem chromatigraphischen Matrerial durchgeführt wird umfassend (i) eine Probenaufnahmezone, (ii) eine Trennzone mit Bindungsbereich in welchem eine oder mehrere sequenzspezifische Nukleinsäuresonden immobilisiert sind und (iii) eine hinter der Trennzone mit Bindungsbereich liegende Flüssigkeit absorbierende Zone, und wobei die sequenzspezifischen Nukleinsäuresonden über einen Polymerlinker immobilisiert sind und wobei das Verfahren folgende Schritte umfasst:
a) die nachzuweisende Nukleinsäure wird im Falle doppelsträngiger Nukleinsäuren denaturiert und anschließend neutralisiert,
b) die nachzuweisende Nukleinsäure wird auf die Probenaufnahmezone in einem Laufpuffer, welcher mild denaturierende Agenzien enthält, aufgetragen,
c) die nachzuweisende Nukleinsäure bewegt sich von der Probenaufnahmezone in Richtung Flüssigkeit absorbierende Zone,
d) die nachzuweisende Nukleinsäure wird im Bindungsbereich der Trennstrecke mit der sequenzspezifischen Nukleinsäuresonde in Kontakt gebracht und hybridisiert an die sequenzspezifische Nukleinsäuresonde
e) die nachzuweisende Nukleinsäure beziehungsweise die Hybridisierung der nachzuweisenden Nukleinsäure an die sequenzspezifische Nukleinsäuresonde wird detektiert über eine Markierung, die an der nachzuweisenden Nukleinsäure angebracht ist oder über die Detektion einer Markierung des Nukleinsäuredoppelstranges.

9. Verfahren nach Anspruch 6 bis 8, wobei der *Staphylococcus aureus* Stamm der identifiziert wird, ausgewählt ist aus der Gruppe umfassend, die epidemischen Stämme:
a) Berlin epidemic strain MRSA Nr. 1417/97 und Nr. 809/96 (internationale Nr. ST 45);
b) Southern German epidemic strain MRSA Nr. 1155/98-2, Nr. 131/98; Nr. 2594/97 (internationale Nr. ST 228);
c) Hannover area epidemic strain MRSA Nr. 872/98 (internationale Nr. ST 254);
d) Northern German epidemic strain MRSA Nr. 134/93, Nr. 1450/94, Nr. 234/95, Nr. 406/98 (internationale Nr. ST 247);
e) Barnim epidemic strain MRSA Nr. 1678-96, Nr. 1293/00, Nr. 152-00, 633/00 (internationale Nr. ST 22) und;
f) Rhine Hesse epidemic strain MRSA Nr. 21663, Nr. 2382-00 und Nr. 2410 (internationale Nr. ST 5).

10. Verfahren nach Ansprüchen 6 bis 9, wobei vor der Hybridisierung eine PCR Amplifikation durchgeführt wird, wobei eine Sequenz nach Anspruch 1 oder ein Abschnitt davon amplifiziert wird und, wobei sequenzspezifische Primer angewendet werden.

11. Verfahren nach Anspruch 10 wobei die PCR Amplifikation mit sequenzspezifischen Primern durchgeführt wird, die im wesentlichen aus folgender Nukleotidsequenz bestehen: und wobei optional das PCR-Produkt markiert wird durch die Inkorporation eins markierten Nukleotides, oder das PCR-Produkt nach der Amplifikation vor der Hybridisierung markiert wird, oder optional einer oder beide markiert sind bevor die PCR-Reaktion initiiert wird.

12. Vorrichtung zur Identifikation eines klinisch relevanten *Staphylococcus aureus* Stammes umfassend eine feste Phase, mit einem oder mehreren Nukleinsäuremolekülen nach den Ansprüchen 3 oder 4.

13. Vorrichtung nach Anspruch 12, wobei die feste Phase ausgewählt ist aus der Gruppe, welche Nukleinsäure Array Mebranen, Nitrozellulose, geladene Nitrozellulose, ungeladene Nitrozellulose, Nylonmembranen, geladene Nylonmembranen, ungeladene Nylonmembranen, Glass-Arrays, umfasst.

14. Kit zur Identifikation klinisch relevanter *Staphylococcus aureus* Stämme umfassend ein Nukleinsäuremolekül nach den Ansprüchen 3 bis 5 oder eine Vorrichtung nach den Ansprüchen 12 bis 13.

## Revendications

1. Molécule d'acide nucléique qui est choisie dans l'ensemble des SEQ ID NOS: 1, 2, 3, 4, 5, 6, 7, 8, 9, 10, 11, 12, 13, 14, 15, 16, 17, 18, 19 et 20 ou complément inverse de celle-ci.

2. Molécule d'acide nucléique de la revendication 1, qui est un ADN génomique, un ADNc, un produit de PCR ou un ARN.

3. Oligonucléotide ou molécule comprenant un oligonucléotide, pour la détection d'une des molécules d'acide nucléique selon les revendications 1 ou 2, choisi dans l'ensemble de séquences nucléotidiques présentées dans SEQ ID NOS: 21, 22, 23, 24, 25, 26, 27, 28 et 29 ou la séquence complémentaire inverse de celui-ci.

4. Oligonucléotide selon la revendication 3, comprenant en plus une modification chimique choisie dans l'ensemble comprenant une modification qui sert à immobiliser l'oligonucléotide et une modification qui sert à marquer l'oligonucléotide.

5. Oligonucléotide selon les revendications 3 ou 4, lequel oligonucléotide est immobilisé sur une phase solide.

6. Procédé d'identification d'une souche de *Staphylococcus aureus* cliniquement pertinente comprenant au moins l'étape de détection d'une molécule d'acide nucléique selon la revendication 1, au moyen d'une hybridation avec une sonde spécifique d'une séquence dans des conditions stringentes.

7. Procédé de la revendication 6, dans lequel la sonde spécifique d'une séquence est un oligonucléotide selon les revendications 3 à 5.

8. Procédé selon la revendication 6 ou 7, dans lequel l'hybridation est effectuée avec un matériau chromatographique qui comprend (i) une zone de réception d'échantillon, (ii) une zone de séparation incluant une zone de liaison, dans laquelle un ou plusieurs acides nucléiques spécifiques d'une séquence sont immobilisés, (ii) et une zone qui absorbe un liquide en aval de la zone de séparation incluant une zone de liaison, (iii) et les sondes d'acide nucléique spécifiques d'une séquence sont immobilisées par l'intermédiaire d'un lieur polymère, lequel procédé comprend les étapes suivantes :
a) dénaturation, dans le cas d'acides nucléiques bicaténaires, de l'acide nucléique à détecter, puis sa neutralisation,
b) application de l'acide nucléique à détecter sur la zone de réception d'échantillons dans un tampon de migration qui contient des agents dénaturants doux,
c) déplacement de l'acide nucléique de la zone de réception d'échantillons dans la direction de la zone d'absorption du liquide,
d) contact de l'acide nucléique à détecter dans la zone de liaison de la piste de séparation avec la sonde d'acide nucléique spécifique d'une séquence et hybridation de celui-ci avec la sonde d'acide nucléique spécifique d'une séquence,
e) détection de l'acide nucléique ou de l'hybridation de l'acide nucléique avec la sonde d'acide nucléique spécifique d'une séquence, par l'intermédiaire d'un marqueur qui est attaché à l'acide nucléique à détecter ou par l'intermédiaire de la détection d'un marqueur du double brin d'acide nucléique.

9. Procédé des revendications 6 à 8, dans lequel la souche de *Staphylococcus aureus* qui doit être identifiée est choisie dans l'ensemble comprenant les souches épidémiques suivantes :
a) la souche épidémique berlinoise MRSA no. 1417/97 et no. 809/96 (no. international ST 45) ;
b) la souche épidémique d'Allemagne du sud MRSA no. IISS/98-2, no. 131/98 ; no. 2594/97 (no. international ST 228) ;
c) la souche épidermique de la zone de Hanovre MRSA no. 872/98 (no. international ST 254) ;
d) la souche épidémique d'Allemagne du nord MRSA no. 134/93, no. 1450/94, no. 234/95, no. 406/98 (no. international ST 247) ;
e) la souche épidémique de Barnim MRSA no. 1678-96, no. 1293/00, No. 152-00, 633/00 (no. international ST 22) et ;
f) la souche épidémique de la Hesse rhénane MRSA no. 21663, no. 2382-00 et no. 2410 (no. international ST 5).

10. Procédé selon les revendications 6 à 9, dans lequel, avant hybridation, une réaction d'amplification par PCR est effectuée, dans laquelle la séquence selon la revendication 1 ou l'une de ses portions est amplifiées et des amorces spécifiques de la séquence sont appliquées.

11. Procédé selon la revendication 10, dans lequel l'amplification par PCR est effectuée avec des amorces spécifiques de séquences consistant essentiellement en la séquence nucléotidique suivante : et éventuellement le produit de PCR est marqué pendant la réaction d'amplification par incorporation d'un nucléotide marqué, ou le produit de PCR est marqué après la réaction de PCR avant l'hybridation et éventuellement l'une ou des deux amorces sont marquées avant l'initiation de la réaction de PCR.

12. Dispositif pour identifier une souche de *Staphylococcus aureus* cliniquement pertinente comprenant une phase solide avec une ou plusieurs molécules d'acide nucléique immobilisées selon les revendications 3 ou 4.

13. Dispositif selon la revendication 12, dans lequel la phase solide est choisie dans l'ensemble comprenant un réseau d'acide nucléique, une membrane, de la nitrocellulose, de la nitrocellulose chargée, de la nitrocellulose non chargée, une membrane de nylon, une membrane de nylon chargée, une membrane de nylon non chargée, un réseau de verre.

14. Kit pour identifier une souche de *Staphylococcus aureus* cliniquement pertinente comprenant une molécule d'acide nucléique selon la revendications 3 à 5, ou un dispositif selon les revendications 12 à 13.


### SEQUENCE LISTING

<110> Federal Republic of Germany represented by the Ministry of Health and Social Security, the latter represented by the President of the Robert Koch Institutes, Nordufer 20, 13353 Berlin, Germany
<120> Substances, Sequences and methods for identifying epidemic methicillin resistant Staphylococcus aureus strains
<130> FBP 13143
<160> 31
<170> PatentIn version 3.1
<210> 1
   <211> 268
   <212> DNA
   <213> Staphylococcus aureus
<400> 1
   caagcaccaa aagaggaaga cggcaacaaa cctggtaaag aagacaacaa aaaacctggc     60
   aaagaagacg gcaacaaacc tggtaaagaa gacaacaaca aacctggtaa agaagacaac    120
   aacaaacctg gtaaagaaga cggcaacaag cctggtaaag aagacaacaa aaaacctggt    180
   aaagaagacg gcaacaaacc tggtaaagaa gacaacaaaa aacctggtaa agaagacggc    240
   aacggagtac atgtcgttaa acctggtg                                       268
<210> 2
   <211> 268
   <212> DNA
   <213> Staphylococcus aureus
<400> 2
   caagcaccaa aagaggaaga cggcaacaaa cctggtaaag aagacaacaa aaaacctggc     60
   aaagaagacg gcaacaaacc tggtaaagaa gacaacaaca aacctggtaa agaagacaac    120
   aacaaacctg gtaaagaaga cggcaacaag cctggtaaag aagacaacaa aaaacctggt    180
   aaagaagacg gcaacaaacc tggtaaagaa gacaacaaaa aacctggtaa agaagacggc    240
   aacggagtac atgtcgttaa acctggtg                                       268
<210> 3
   <211> 295
   <212> DNA
   <213> Staphylococcus aureus
<400> 3
   caagcaccaa aagaggaaga caacaaaaaa cctggtaaag aagacggcaa caaaaaacct     60
   ggtaaagaag acggcaacaa gcctggtaaa gaagacaaca aaaaacctgg taaagaagac    120
   ggcaacaagc ctggtaaaga agacaacaac aaacctggca aagaagacgg caacaagcct    180
   ggtaaagaag acaacaacaa gcctggtaaa gaagacggca acaagcctgg taaagaagac    240
   ggcaacaaac ctggtaaaga agacggcaac ggagtacatg tcgttaaacc tggtg          295
<210> 4
   <211> 295
   <212> DNA
   <213> Staphylococcus aureus
<400> 4
   caagcaccaa aagaggaaga caacaaaaaa cctggtaaag aagacggcaa caaaaaacct     60
   ggtaaagaag acggcaacaa gcctggtaaa gaagacaaca aaaaacctgg taaagaagac    120
   ggcaacaagc ctggtaaaga agacaacaac aaacctggca aagaagacgg caacaagcct    180
   ggtaaagaag acaacaacaa gcctggtaaa gaagacggca acaagcctgg taaagaagac    240
   ggcaacaaac ctggtaaaga agacggcaac ggagtacatg tcgttaaacc tggtg          295
<210> 5
   <211> 295
   <212> DNA
   <213> Staphylococcus aureus
<400> 5
   caagcaccaa aagaggaaga caacaaaaaa cctggtaaag aagacggcaa caaaaaacct     60
   ggtaaagaag acggcaacaa gcctggtaaa gaagacaaca aaaaacctgg taaagaagac    120
   ggcaacaagc ctggtaaaga agacaacaac aaacctggca aagaagacgg caacaagcct    180
   ggtaaagaag acaacaacaa gcctggtaaa gaagacggca acaagcctgg taaagaagac    240
   ggcaacaaac ctggtaaaga agacggcaac ggagtacatg tcgttaaacc tggtg          295
<210> 6
   <211> 292
   <212> DNA
   <213> Staphylococcus aureus
<400> 6
   caagcaccaa aagaggaaga caataacaag cctggcaaag aagacaacaa caagcctggt      60
   aaagaagaca acaacaagcc tggtaaagaa gacaacaaaa aacctggtaa agaagacggc     120
   aacaagcctg gcaaagaaga tggcaacaag cctggtaaag aagacaacaa aaaacctggt     180
   aaagaagacg gcaacaagcc tggcaaagaa gatggcaaca agcctggcaa agaagatggc     240
   aacaaacctg gtaaagaaga tggtaacgga gtacatgtcg ttaaacctgg tg             292
<210> 7
   <211> 316
   <212> DNA
   <213> Staphylococcus aureus
<400> 7
   caagcaccaa aagaggaaga caataacaag cctggcaaag aagacaataa caagcctggc      60
   aaagaagaca acaacaagcc tggcaaagaa gacaacaaca agcctggtaa agaagacaac     120
   aacaagcctg gcaaagaaga cggcaacaag cctggtaaag aagacaacaa aaaacctggt     180
   aaagaagatg gcaacaagcc tggtaaagaa gacaacaaaa aacctggtaa agaagacggc     240
   aacaagcctg gcaaagaaga tggcaacaaa cctggtaaag aagatggtaa cggagtacat     300
   gtcgttaaac ctggtg                                                     316
<210> 8
   <211> 316
   <212> DNA
   <213> Staphylococcus aureus
<400> 8
   caagcaccaa aagaggaaga caataacaag cctggcaaag aagacaataa caagcctggc      60
   aaagaagaca acaacaagcc tggcaaagaa gacaacaaca agcctggtaa agaagacaac     120
   aacaagcctg gcaaagaaga cggcaacaag cctggtaaag aagacaacaa aaaacctggt     180
   aaagaagatg gcaacaagcc tggtaaagaa gacaacaaaa aacctggtaa agaagacggc     240
   aacaagcctg gcaaagaaga tggcaacaaa cctggtaaag aagatggtaa cggagtacat     300
   gtcgttaaac ctggtg                                                     316
<210> 9
   <211> 316
   <212> DNA
   <213> Staphylococcus aureus
<400> 9
   caagcaccaa aagaggaaga caataacaag cctggcaaag aagacaataa caagcctggc     60
   aaagaagaca acaacaagcc tggcaaagaa gacaacaaca agcctggtaa agaagacaac    120
   aacaagcctg gcaaagaaga cggcaacaag cctggtaaag aagacaacaa aaaacctggt    180
   aaagaagatg gcaacaagcc tggtaaagaa gacaacaaaa aacctggtaa agaagacggc    240
   aacaagcctg gcaaagaaga tggcaacaaa cctggtaaag aagatggtaa cggagtacat    300
   gtcgttaaac ctggtg                                                    316
<210> 10
   <211> 316
   <212> DNA
   <213> Staphylococcus aureus
<400> 10
   caagcaccaa aagaggaaga caataacaag cctggcaaag aagacaataa caagcctggc     60
   aaagaagaca acaacaagcc tggcaaagaa gacaacaaca agcctggtaa agaagacaac    120
   aacaagcctg gcaaagaaga cggcaacaag cctggtaaag aagacaacaa aaaacctggt    180
   aaagaagatg gcaacaagcc tggtaaagaa gacaacaaaa aacctggtaa agaagacggc    240
   aacaagcctg gcaaagaaga tggcaacaaa cctggtaaag aagatggtaa cggagtacat    300
   gtcgttaaac ctggtg                                                    316
<210> 11
   <211> 391
   <212> DNA
   <213> Staphylococcus aureus
<400> 11
   ggcaacaaac ctggcaaaga agacggcaac aaacctggca aagaagacaa caacaaacct     60
   ggtaaagaag acggcaacaa acctggcaaa gaagatggca acaaacctgg caaagaagac    120
   aacaaaaagc ctagcaaaga agacggcaac aagcctggta aagaagatgg caacaaacct    180
   ggcaaagaag acaacaaaaa gcctagcaaa gaagacggca acaagcctgg taaagaagat    240
   ggcaacaaac ctggtaaaga agacggcaac aagcctggta aagaagatgg caacaaacct    300
   ggtaaagaag acggcaacaa acctggtaaa gaagatggta acaaacctgg caaagaagac    360
   ggcaacgggg tacatgtcgt taaacctggt g                                   391
<210> 12
   <211> 436
   <212> DNA
   <213> Staphylococcus aureus
<400> 12
   caagcaccaa aagaggaaga caacaaaaaa cctggtaaag aagacggcaa caaacctggc     60
   aaagaagacg gcaacaaacc tggcaaagaa gacaacaaca aacctggtaa agaagacggc    120
   aacaaacctg gcaaagaaga tggcaacaaa cctggcaaag aagacaacaa aaagcctagc    180
   aaagaagacg gcaacaagcc tggtaaagaa gatggcaaca aacctggcaa agaagacaac    240
   aaaaagccta gcaaagaaga cggcaacaag cctggtaaag aagatggcaa caaacctggt    300
   aaagaagacg gcaacaagcc tggtaaagaa gatggcaaca aacctggtaa agaagacggc    360
   aacaaacctg gtaaagaaga tggtaacaaa cctggcaaag aagacggcaa cggggtacat    420
   gtcgttaaac ctggtg                                                    436
<210> 13
   <211> 436
   <212> DNA
   <213> Staphylococcus aureus
<400> 13
   caagcaccaa aagaggaaga caacaaaaaa cctggtaaag aagacggcaa caaacctggc     60
   aaagaagacg gcaacaaacc tggcaaagaa gacaacaaca aacctggtaa agaagacggc    120
   aacaaacctg gcaaagaaga tggcaacaaa cctggcaaag aagacaacaa aaagcctagc    180
   aaagaagacg gcaacaagcc tggtaaagaa gatggcaaca aacctggcaa agaagacaac    240
   aaaaagccta gcaaagaaga cggcaacaag cctggtaaag aagatggcaa caaacctggt    300
   aaagaagacg gcaacaagcc tggtaaagaa gatggcaaca aacctggtaa agaagacggc    360
   aacaaacctg gtaaagaaga tggtaacaaa cctggcaaag aagacggcaa cggggtacat    420
   gtcgttaaac ctggtg                                                    436
<210> 14
   <211> 436
   <212> DNA
   <213> Staphylococcus aureus
<400> 14
   caagcaccaa aagaggaaga caacaaaaaa cctggtaaag aagacggcaa caaacctggc 60
   aaagaagacg gcaacaaacc tggcaaagaa gacaacaaca aacctggtaa agaagacggc 120
   aacaaacctg gcaaagaaga tggcaacaaa cctggcaaag aagacaacaa aaagcctagc 180
   aaagaagacg gcaacaagcc tggtaaagaa gatggcaaca aacctggcaa agaagacaac 240
   aaaaagccta gcaaagaaga cggcaacaag cctggtaaag aagatggcaa caaacctggt 300
   aaagaagacg gcaacaagcc tggtaaagaa gatggcaaca aacctggtaa agaagacggc 360
   aacaaacctg gtaaagaaga tggtaacaaa cctggcaaag aagacggcaa cggggtacat 420
   gtcgttaaac ctggtg 436
<210> 15
   <211> 244
   <212> DNA
   <213> Staphylococcus aureus
<400> 15
   caagcaccaa aagaggaaga caacaaaaaa cctggtaaag aagacggcaa caagcctggt 60
   aaagaagaca acaacaaacc tggcaaagaa gacggcaaca agcctggtaa agaagacaac 120
   aacaagcctg gtaaagaaga cggcaacaag cctggtaaag aagacggcaa caagcctggt 180
   aaagaagacg gcaacaaacc tggtaaagaa gacggcaacg gagtacatgt cgttaaacct 240
   ggtg 244
<210> 16
   <211> 268
   <212> DNA
   <213> Staphylococcus aureus
<400> 16
   caagcaccaa aagaggaaga caacaaaaaa cctggtaaag aagacggcaa caaacctggc 60
   aaagaagacg gcaacaagcc tggtaaagaa gacaacaaaa aacctggtaa agaagacggc 120
   aacaagcctg gtaaagaaga caacaacaaa cctggcaaag aagacggcaa caagcctggt 180
   aaagaagacg gcaacaagcc tggtaaagaa gacggcaaca aacctggtaa agaagacggc 240
   aacggagtac atgtcgttaa acctggtg 268
<210> 17
   <211> 268
   <212> DNA
   <213> Staphylococcus aureus
<400> 17
   caagcaccaa aagaggaaga caacaaaaaa cctggtaaag aagacggcaa caaacctggc     60
   aaagaagacg gcaacaagcc tggtaaagaa gacaacaaaa aacctggtaa agaagacggc    120
   aacaagcctg gtaaagaaga caacaacaaa cctggcaaag aagacggcaa caagcctggt    180
   aaagaagaca acaacaagcc tggtaaagaa gacggcaaca agcctggtaa agaagacggc    240
   aacggagtac atgtcgttaa acctggtg                                       268
<210> 18
   <211> 364
   <212> DNA
   <213> Staphylococcus aureus
<400> 18
   caagcaccaa aagaggaaga caataacaag cctggcaaag aagacaacaa caagcctggt     60
   aaagaagaca acaacaagcc tggcaaagaa gacggcaaca agcctggtaa agaagacaac    120
   aaaaaacctg gtaaagaaga tggcaacaag cctggtaaag aagacaacaa aaaacctggt    180
   aaagaagacg gcaacaagcc tggcaaagaa gatggcaaca agcctggtaa agaagacaac    240
   aaaaaacctg gtaaagaaga cggcaacaag cctggcaaag aagatggcaa caagcctggc    300
   aaagaagatg gcaacaaacc tggtaaagaa gatggtaacg gagtacatgt cgttaaacct    360
   ggtg                                                                 364
<210> 19
   <211> 364
   <212> DNA
   <213> Staphylococcus aureus
<400> 19
   caagcaccaa aagaggaaga caataacaag cctggcaaag aagacaacaa caagcctggt     60
   aaagaagaca acaacaagcc tggcaaagaa gacggcaaca agcctggtaa agaagacaac    120
   aaaaaacctg gtaaagaaga tggcaacaag cctggtaaag aagacaacaa aaaacctggt    180
   aaagaagacg gcaacaagcc tggcaaagaa gatggcaaca agcctggtaa agaagacaac    240
   aaaaaacctg gtaaagaaga cggcaacaag cctggcaaag aagatggcaa caagcctggc    300
   aaagaagatg gcaacaaacc tggtaaagaa gatggtaacg gagtacatgt cgttaaacct    360
   ggtg                                                                 364
<210> 20
   <211> 364
   <212> DNA
   <213> Staphylococcus aureus
<400> 20
   caagcaccaa aagaggaaga caataacaag cctggcaaag aagacaacaa caagcctggt     60
   aaagaagaca acaacaagcc tggcaaagaa gacggcaaca agcctggtaa agaagacaac    120
   aaaaaacctg gtaaagaaga tggcaacaag cctggtaaag aagacaacaa aaaacctggt    180
   aaagaagacg gcaacaagcc tggcaaagaa gatggcaaca agcctggtaa agaagacaac    240
   aaaaaacctg gtaaagaaga cggcaacaag cctggcaaag aagatggcaa caagcctggc    300
   aaagaagatg gcaacaaacc tggtaaagaa gatggtaacg gagtacatgt cgttaaacct    360
   ggtg                                                                 364
<210> 21
   <211> 24
   <212> DNA
   <213> Staphylococcus aureus
<400> 21
   caacaacaag cctggtaaag aaga    24
<210> 22
   <211> 24
   <212> DNA
   <213> Staphylococcus aureus
<400> 22
   ggcaacaaaa aacctggtaa agaa    24
<210> 23
   <211> 24
   <212> DNA
   <213> Staphylococcus aureus
<400> 23
   aataacaagc ctggcaaaga agac    24
<210> 24
   <211> 24
   <212> DNA
   <213> Staphylococcus aureus
<400> 24
   ggcaacaaac ctggcaaaga agac    24
<210> 25
   <211> 24
   <212> DNA
   <213> Staphylococcus aureus
<400> 25
   aagcctagca aagaagacgg caac 24
<210> 26
   <211> 20
   <212> DNA
   <213> Staphylococcus aureus
<400> 26
   gtaaagaagg caacggagta 20
<210> 27
   <211> 21
   <212> DNA
   <213> Staphylococcus aureus
<400> 27
   aaaacctggc aaagaagacg g 21
<210> 28
   <211> 26
   <212> DNA
   <213> Staphylococcus aureus
<400> 28
   aacaacaaac ctggtaaaga agacaa 26
<210> 29
   <211> 21
   <212> DNA
   <213> Staphylococcus aureus
<400> 29
   ggaagacggc aacaaacctg g 21
<210> 30
   <211> 18
   <212> DNA
   <213> Staphylococcus aureus
<400> 30
   caagcaccaa aagaggaa 18
<210> 31
   <211> 18
   <212> DNA
   <213> Staphylococcus aureus
<400> 31
   caccaggttt aacgacat 18